# EUROPEAN PATENT SPECIFICATION

(11) **EP 0 651 806 B1**
(45) Date of publication and mention of the grant of the patent: **20.05.1998**
(21) Application number: 93915919.0
(22) Date of filing: 15.07.1993
(51) Int. Cl.: C12N 15/49, A61K 31/70, C07K 14/155, A61K 39/21, C12N 5/10

(54) **ANTI-FELINE IMMUNODEFICIENCY VIRUS (FIV) VACCINES**
Anti-Katze Immundefiziensvirus (FIV) Impfstoffe
VACCINS CONTRE LE VIRUS DE L'IMMUNODEFICIENCE ANTI-FELINE (VIF)

(30) Priority: 17.07.1992 GB 9215232
(43) Date of publication of application: 10.05.1995
(73) Proprietor: MALLINCKRODT VETERINARY, INC., Mundelein, Illinois 60060 (US)
(72) Inventor: OSTERHAUS, Albertus Dominicus Marcellinus Erasmus, NL-3981 CH Bunnik (NL); SIEBELINK, Cornelis Herman Johannes, NL-3833 GD Leusden (NL)
(74) Representative: Dzieglewska, Hanna Eva
(86) International application number: EP9301860
(87) International publication number: WO9402612

(56) References cited:
- WO-A-92/09632
- WO-A-92/15684
- VIRUS RESEARCH, Vol. 21, No. 1, January 1991, Amsterdam, NL, pages 53-63, S. MORIKAWA et al., "Identification of Conserved and Variable Regions in the Envelope Glycoprotein Sequences of Two Feleine Immunodeficiency Viruses Isolated in Zurich, Switzerland".
- JOURNAL OF VIROLOGY, Vol. 67, No. 2, February 1993, pages 664-672, G. PANCINO et al., "B Epitopes and Selection Pressures in Feline Immunodeficiency Virus Envelope Glycoproteins".

## Description

The present invention relates to feline immunodeficiency virus (FIV) and to the development of vaccines for use in protecting cats and kittens against FIV infection.

FIV is a recently discovered T-lymphotropic lentivirus which infects cats to produce an AIDS-like syndrome. FIV, while exhibiting morphological and pathological similarity, has however been shown to be antigenically distinct from the human immunodeficiency virus (HIV) (Pederson et al., Science 235: 790-793, 1987). Infected cats and kittens show a generally debilitating AIDS-like disease with intermittent symptoms (eg. lymphadenopathy, leucopenia, anaemia and secondary opportunistic infection) leading ultimately to death.

Epidemiological studies have shown FIV infection to be widespread worldwide and the disease is rapidly acquiring significant clinical importance from a veterinary point of view. Efforts have accordingly recently begun to be directed to the development of vaccines against FIV but whilst preliminary results with vaccines based on fixed infected whole cells or whole inactivated virus look promising (Yamamoto et al., AIDS research and human retroviruses, 7(11): 911-922, 1991), there are as yet no reports in the literature of successful immunisation of cats against FIV infection using sub-unit or peptide-based vaccines. No commercial vaccines are currently available.

Despite its ubiquitous presence, FIV does not appear to be capable of cat to human transmission. Nonetheless, FIV shares with HIV and other mammalian lentiviruses similarities in genome organisation, biological properties, the propensity for persistent infection in the natural host, with its concomitant pathological manifestations (eg. decline in CD4+ lymphocytes, both in vivo and in vitro, gradual loss of immune function, and opportunistic infection). Thus the development of experimental models of non-human lentivirus infections, such as FIV, may facilitate the design of vaccine and therapeutic strategies for HIV infection of humans.

The molecular structure of FIV, in terms of genome organisation and nucleotide sequence, has been studied (Talbot et al., PNAS, 86: 5743-5747, 1989; Olmstead et al., PNAS 86: 8088-8092, 1989) but as yet there are few reports of the identification of antigenically important regions and no reports of specific regions which may contain epitopes useful for the development of neutralising vaccines or drug therapies.

Replication competent molecular clones of FIV have recently become available (Siebelink et al., J. Virol. 66(3): 1091-1097, 1992). Molecular cloning and sequence analysis have shown that viral isolates obtained from different geographical regions are heterogenous (Morikawa et al., Virus Research, 21: 53-63, 1991) whereas clones obtained from the same isolate (Olmstead et al., Talbot et al., supra) or cloned directly ex vivo from the same cat (Siebelink et al., 1992, supra) are highly homogenous. Genomic diversity in the human counterpart HIV-1 reflects diversity in the biological properties of different HIV-1 isolates (eg. cytopathic changes in infected cells, cell tropism, viral replication and virus neutralisation) and as a result many different approaches have had to be investigated in the search for an effective HIV vaccine or therapy, including for example investigation of the effects of neutralising or opsonizing antibodies, antibody mediated cellular cytotoxicity, complement mediated cytolysis, cytotoxic T lymphocytes, and the production of interleukins and other immune effector molecules and the role they may play in the elimination of virus. As is clear from contemporary literature reports, much of this intensive activity has yet to prove commercially fruitful and an effective commercial HIV vaccine is far from the horizon. Based on this, similar difficulties may have been expected in the search for an effective FIV vaccine or therapy.

We have now found however that particular regions of the FIV envelope protein may be of importance for recognition of FIV by neutralising antibodies and these particular regions, located in the hypervariable regions V4 and V5, are now proposed, according to the present invention, as the basis for a vaccine against FIV infection in cats. In particular we have identified neutralisation sites between residues 483 to 567 of the FIV envelope protein.

According to one aspect the present invention thus provides use of a nucleotide sequence containing a part of the FIV env gene, said part consisting essentially of a nucleotide sequence corresponding to the region of the FIV env gene encoding residues 483 to 567 of the FIV envelope protein or a portion thereof encoding an FIV-combatting product or sequence which is degenerate, has greater than 60% sequence homology therewith, in the preparation of a composition for combatting FIV, preferably a vaccine against FIV infection in animals, e.g. mammals, ie. a vaccine composition for stimulating an immune response against FIV.

Alternatively viewed, the invention also provides a nucleic acid molecule the nucleotide sequence of which corresponds to the region of the FIV env gene encoding residues 483 to 567 of the FIV envelope protein or a portion thereof encoding an FIV-combatting product or a sequence which is degenerate, has greater than 60% sequence homology therewith or which hybridises under conditions of high stringency with any such aforesaid sequence for use in the preparation of a composition for combatting FIV, preferably a vaccine against FIV infection in animals.

A further aspect of the invention provides a nucleic acid molecule comprising a first nucleotide sequence consisting of a sequence corresponding to the region of the FIV env gene encoding residues 483 to 567 of the FIV envelope protein or a portion thereof encoding an FIV-combatting product or a sequence which has greater than 60% sequence homology therewith, together with at least one additional nucleotide sequence flanking said first nucleotide sequence, the additional nucleotide sequence comprising no more than 150 bases, preferably no more than 100 eg. no more than 50 bases.

Nucleic acid molecules according to the invention may be single or double stranded DNA, cDNA or RNA, and conveniently take the form of a recombinant nucleic acid molecule, preferably a recombinant DNA molecule.

The additional flanking sequences in the nucleic acid molecule according to the invention may be derived from the FIV env gene itself, from other regions of FIV DNA or from heterologous sources, and may be coding or noncoding. In a preferred aspect the flanking sequences may contain one or more restriction sites.

As mentioned above, variations in the env coding region may occur between different strains or serotypes of FIV, isolates of different geographical origin or even between different isolates from the same host and such variations in the region of the FIV env gene encoding residues 483-567 of the FIV envelope protein, which express as products capable of combatting FIV eg. by stimulating an immune response against FIV, are included in the scope of this invention. Different FIV isolates and sequence divergence between them are described for example by Rigby et al., in Journal of General Virology (1993), 74, 425-436.

"Substantially homologous" as used herein includes those sequences having a sequence homology of approximately 60% or more, eg. 70% or 80% of more, and also functionally-equivalent allelic variants and related sequences modified by single or multiple base substitution, addition and/or deletion. By "functionally equivalent" is meant nucleotide sequences which encode immunoreactive polypeptides eg. polypeptides which are recognised by neutralising antibodies generated by the host, or which otherwise are capable of combatting the FIV virus.

Methods for producing such derivative related sequences, for example by site-directed mutagenesis, random mutagenesis, or enzymatic cleavage and/or ligation of nucleic acids are well known in the art, as are methods for determining whether the thus-modified nucleotide sequence has significant homology to the subject sequence, for example by hybridisation.

Provision of a nucleotide sequence according to the invention thus enables recombinant fragments of immunogenically important regions of the FIV envelope protein to be obtained in significant quantities, heretofore unavailable, thereby permitting the development of anti-FIV vaccines and therapies.

In another aspect the present invention thus provides a use of a nucleic acid molecule comprising a nucleotide sequence encoding a polypeptide capable of raising neutralising antibodies against FIV, consisting essentially of one or more antigenic determinant-encoding regions from the regions of the FIV env gene encoding residues 483-567 of the envelope protein for the preparation of a composition for combatting FIV, preferably a vaccine composition.

The present invention also provides peptides, suitable for use in vaccines, which present an epitope comprising all or a portion of amino acid residues 483-567 of the FIV envelope protein, or equivalent amino acid residues of another FIV protein. These amino acids may be replaced by other amino acids which do not affect the antigenicity of the peptides. The peptides may be provided as a conjugate linked to a physiologically acceptable carrier, or they may comprise a carrier sequence.

The identification, according to the present invention, of a neutralisation site, that is a region of the FIV env gene encoding a part of the envelope glycoprotein which is recognised by host neutralising antibodies, is of great importance in the development of an anti-FIV vaccine, since being a retrovirus, FIV can integrate into the genome of infected host cells in which it can hide and/or escape from the host immune system, and thus a rapid neutralisation of the virus, before it can infect host cells, is desirable.

The neutralisation site(s) between residues 483 to 567 of the FIV envelope protein were identified according to the present invention, by generating so-called escape mutants of the FIV env gene. Such mutants of FIV escape or evade the host immune response by mutating in regions recognised by host neutralising antibodies. Escape mutants have been described for a number of lentiviruses (eg. visna virus - see Narayan et al., J. Virol. 32: 1045-1050, 1981) but so far no escape mutants have been described for FIV, and no neutralising epitopes have previously been identified.

As mentioned above, molecular clones of FIV are available and the nucleotide sequences of such clones have been published (see for example Siebelink 1992 supra). We have generated escape mutants of one such clone, 19k1 (Siebelink, supra) by culturing viral progeny of this clone in the presence of serum from a specific pathogen free (SPF) cat which had been infected with this clone. By sequencing the envelope gene of such escape mutants and comparing with the sequence of the parent clone, the regions of mutation, and thus the sites in which neutralisation-resistance has developed (the neutralisation site) can be identified. Escape mutants in the V4 and V5 hypervariable region-encoding portion of the FIV env gene, and particularly in the regions encoding residues 483 to 567 of the envelope protein, form a further aspect of the present invention.

In particular, as will be described in more detail below, escape mutants have been generated, in the regions encoding residue 483 and residues 549 to 567 of the FIV env gene. These regions are preferred according to the present invention. Whilst we do not wish to be bound by theoretical considerations, we believe that the region of the FIV env protein spanning residues 483 to 567 may contain or may contribute to one or more antigenically important epitopic sites. Thus, in addition to linear, continuous epitopes it is possible that important epitope(s) in this region are discontinuous, that is they may comprise amino acid residues from different regions of the protein, and that such epitopes include amino acid residues contributed by the region 483 to 567 in the FIV env gene, in particular residue 483 and other residues in the region 549 to 567.

Thus viewed from a further aspect, the present invention provides a nucleic acid molecule as hereinbefore described wherein said nucleotide sequence encodes an FIV-combatting product and (i) includes codons of the FIV env gene encoding residues selected from residue 483 and residues 549 to 567 of the FIV envelope protein in discontinuous arrangement; or (ii) includes the codon of the FIV env gene encoding residue 483 of the FIV envelope protein; or (iii) consists of the sequence of the FIV env gene encoding residues 549 to 567 of the FIV envelope protein.

The nucleotide sequences according to the invention as defined above can be expressed, in appropriate expression systems well known in the art, as synthetic polypeptides containing a part of the FIV envelope protein, said part consisting essentially of an amino acid sequence corresponding to the region of the FIV envelope protein spanning residues 483-567 or a portion thereof which is an FIV-combatting product, or functionally-equivalent variants thereof. Such synthetic polypeptides and vaccine compositions comprising them, together with pharmaceutically acceptable carriers and/or adjuvants, form further aspects of the present invention.

"Functionally equivalent" as used above in relation to the polypeptide amino acid sequences defines polypeptides related to or derived from the native FIV envelope protein where the amino acid sequence has been modified by single or multiple amino acid substitution, addition or deletion, and also sequences where the amino acids have been chemically modified, including by glycosylation or deglycosylation, but which nonetheless retain immunogenic or other FIV-combatting activity eg. are capable of raising neutralising antibodies in the host.

Such functionally-equivalent variants may occur as natural biological variants or may be prepared using known techniques, for example functionally equivalent recombinant polypeptides may be prepared using the known techniques of site-directed mutagenesis, random mutagenesis, or enzymatic cleavage and/or ligation of amino acids.

The term "polypeptide" as used herein defines both long chain polypeptides and shorter peptide sequences.

As mentioned above, modification of the amino acid sequences to obtain functionally-equivalent variant sequences may be by amino acid substitution, as long as the immunogenicity of the polypeptide is not affected. Thus for example, an amino acid may be replaced by another which preserves the physicochemical character of the polypeptide or its epitope(s) eg. in terms of charge density, hydrophilicity/hydrophobicity, size and configuration and hence preserve the immunological structure. For example A may be replaced by G or vice versa, V by A, L or G; K by R; S by T or vice versa; E by D or vice versa; and Q by N or vice versa. Generally, the substituting amino acid has similar properties eg. hydrophobicity, hydrophilicity, electronegativity, bulky side chains etc. to the amino acid being replaced.

"Addition" variants include amino and/or carboxyl terminal fusions, for example by addition of amino acid sequences of up to 300 eg. up to 200 or 100 residues, as well as intrasequence insertions of single or multiple amino acids. Amino acid sequences added may be those provided in the corresponding positions in the FIV envelope protein of other variants or other amino acids, eg. the whole or parts of other polypeptides or proteins. Longer peptides may comprise multiple copies of one or more of the polypeptide sequences.
Alternatively, multiple copies of the polypeptides (e.g. 5 to 20, preferably 8 to 15) may be coupled to a polyamino acid backbone, eg. a polylysine backbone to form so-called multiple antigen peptides (MAPs) as described for example by Tam in PNAS 85: 5400-5413, 1988).

Insertional amino acid sequence variants are those in which one or more amino acid residues are introduced into a predetermined site in the protein although random insertion is also possible with suitable screening of the resulting product. Deletional variants are characterised by the removal of one or more amino acids from the sequence. Preferably, deletions or insertions are made in adjacent pairs eg. a deletion of two residues or insertion of two molecules. In all cases the proviso is that the modification preserves the immunogenicity of the polypeptide.

Exemplary functionally-equivalent variant polypeptides may thus include those displaying at least 50%, eg. at least 60 or 70%, or more preferably greater than 80% amino acid sequence homology. It should be noted however that functionally-equivalent variants, may exhibit overall sequence homology below the given percentages, but still fall within the scope of the present invention where they have conserved regions of homology.

It may in certain cases be convenient to include, where one does not occur naturally, one or more cysteine residues at the termini of the polypeptides, for example to enable specific carrier linkage or to permit disulphide bonding - this may be desirable to enable the polypeptides to mimic antigenic loops such as may appear on the surface of proteins and thereby enhance their immunogenicity.

A fatty acid or hydrophobic tail may also be added to the peptides to enhance immunogenicity and facilitate incorporation into delivery vehicles such as liposomes, novosomes and ISCOMS (Reid, Vaccine 10: 597-601, 1992).

The amino acid residues of the synthetic polypeptides of the invention may be chemically modified, particularly at the ends of the molecule, and may take the form, for example, of amino acid ester or amides. Thus, for example, N- or C-terminal residues, eg. N- or C-terminal, particularly C-terminal cysteine residues may be chemically blocked or protected for example by an acetamido or other protecting group. A wide range of blocking groups are known in the art and may be used, including for example sulphonate, carboxymethyl, carboxamidomethyl, amino ethyl and similar groups.

The polypeptide may be linked to a carrier in order to create a conjugate which is immunogenically active. Any appropriate physiologically acceptable carrier may be employed, for example, a protein such as bovine serum albumin, thyroglobulin, ovalbumin or keyhole limpet hemocyanin. Recently, the concept of presenting multiple copies of viral peptides on the surface of particulate structures in a manner that resembles a virion has received much attention. This can be achieved either by producing virus/peptide chimaeras, eg. with poliovirus, or by linking the peptide to proteins which naturally self assemble into particles such as hepatitis B surface antigen, the Ty protein from yeast, or the core protein from hepatitis virus (HBcAg). The polypeptide may also be linked to other FIV proteins or polypeptides thereby providing both an immunogen and a multivalent vaccine at the same time.

Rather than link a carrier sequence to a peptide in this way, a polypeptide may be prepared which itself incorporates an appropriate carrier sequence ie. as a fusion protein comprising the synthetic polypeptide(s) of the invention or a longer sequence incorporating such a polypeptide linked to a heterologous carrier sequence, as will be described in more detail below. Such carrier proteins are generally chosen so that the resultant product is physiologically acceptable.

Other modifications which may be made to enhance immunogenicity of the synthetic polypeptides include the introduction of, or coupling to longer amino acid sequences containing FIV helper T-cell epitopes. Particular mention may be made in this regard of particulate carrier proteins such as HBcAg which contain helper T-cell epitopes and thus have the dual advantages of a highly immunogenic mode of antigen presentation and the presence of helper T-cell epitopes. Such modifications, and many others which may enhance antigen presentation and/or delivery to the immune system, including those described above are well known in the art and are discussed and reviewed by Francis in Vaccines, Eds. Gregoriadis et al, Plenum Press, New York, p 13-23, 1991; Sci. Progress 74: 115-130, 1990 and by Francis & Clarke in Meth. Enzymol., 178: 659-676, 1989.

The synthetic polypeptides of the invention may be presented as pharmaceutically or physiologically acceptable salts eg. acid addition salts. This may include both organic and inorganic salts such as those prepared for example from acids such as hydrochloric, hydrofluoric, sulfuric, sulfonic, tartaric, fumaric, hydrobromic, glycolic, citric, maleic, phosphoric, succinic, acetic, nitric, benzoic, ascorbic, p-toluenesulfonic, benzene-sulfonic, naphthalenesulfonic, propionic, and the like. Preferably, the acid addition salts are those prepared from hydrochloric acid, acetic acid, or succinic acid. Such salts may be prepared by conventional methods well known to those skilled in the art.

Alternatively the peptide may be converted into a carboxylic acid salt, such as an ammonium or alkali metal salt eg. a sodium, potassium, or lithium salt etc.

As mentioned above, the synthetic FIV polypeptides according to the invention may be used to combat FIV infection in animals, and preferably to stimulate a host immune response against FIV. Such an immune response may comprise elements of both humoral and/or cell-medicated immunity to protect the host from FIV infection and/or kill or inhibit the virus, and may thus for example include the generation of immune effector molecules, antibodies or cells which damage, inhibit or kill the virus. Commonly, such a host-protective immune response may be manifested by the generation of antibodies which are able to neutralise the virus.

The synthetic FIV polypeptides of the invention may exert their host protective effects is by raising neutralising antibodies which inhibit the growth and/or maintenance of the virus. Such neutralising antibodies, which may be mono- or polyclonal, form a further aspect of the invention as do vaccine compositions containing them and their use in the preparation of vaccine compositions for passively immunising hosts against FIV infection. Techniques for obtaining mono- or polyclonal antibodies are well known in the art.

In further aspects the present invention thus provides use of a polypeptide containing a part of the FIV envelope protein, said part consisting essentially of an amino acid sequence corresponding to all or a portion of the region of the FIV envelope protein spanning residues 483 to 567, or a functionally equivalent variant thereof, for the preparation of a composition for combatting FIV, preferably a vaccine composition for stimulating an immune response against FIV in an animal, and a polypeptide as defined above for use in the preparation of a FIV-combatting composition, preferably a vaccine composition for stimulating an immune response against FIV in an animal.

The synthetic polypeptides according to this aspect of the invention may be prepared by expression in a host cell containing a recombinant DNA molecule which comprises a nucleotide sequence as broadly defined above, operatively linked to an expression control sequence, or a recombinant DNA cloning vehicle or vector containing such a recombinant DNA molecule. Alternatively, the polypeptides may be expressed by direct injection of a naked DNA molecule comprising a nucleotide sequence according to the invention into a host cell. Appropriate recombinant DNA technology and expression techniques are described for example by Sambrook et al., 1989 (Molecular cloning, a laboratory manual, 2nd Edition, Cold Spring Harbor Press).

The polypeptides so expressed may be fusion polypeptides comprising all or a portion of the region of the FIV envelope protein spanning residues 483-567 according to the invention, and an additional polypeptide coded for by the DNA of the recombinant molecule fused thereto. This may for example by β-galactosidase, glutathione-S-transferase, yeast Ty particles, hepatitis core antigen or the transmembrane portion of a membrane protein or any other of the polypeptides commonly employed in fusion proteins in the art.

In an alternative form of fusion protein, one or more synthetic polypeptides of the invention may replace one or more antigenic epitopes, or parts thereof, of another protein such as hepatitis core antigen, yeast Ty particles or influenza virus haemagglutonin (HA).

Other aspects of the invention thus include cloning and expression vectors containing DNA comprising nucleotide sequences according to the invention and methods for preparing the nucleic acid molecules of the invention, by inserting the said nucleotide sequences into vector nucleic acid, eg. vector DNA. Such expression vectors include appropriate control sequences such as for example translational (eg. start and stop codes) and transcriptional control elements (eg. promoter-operator regions, ribosomal binding sites, termination stop sequences) linked in matching reading frame with the nucleic acid molecules of the invention.

The invention also includes transformed or transfected prokaryotic or eukaryotic host cells, or transgenic organisms containing a nucleotide sequence according to the invention as defined above, as well as methods for preparing synthetic polypeptides of the invention by culturing host cells containing a nucleic acid molecules as defined above under conditions whereby said polypeptide is expressed and recovering such polypeptide thus produced. As mentioned above, preferred nucleic acid molecules, vectors, host cells and organisms etc. are those which include sequences encoding residues 483 and 549 to 567 of the FIV env protein.

Cloning or expression vectors according to the invention may include plasmids, phage and recombinant viruses, according to techniques well known in the art and may be expressed in a variety of different expression systems cells, including bacterial (eg. E. coli) yeast or mammalian expression systems. To enable appropriate glycosylation of the FIV polypeptides of the invention to take place, expression in eukaryotic, preferably mammalian, systems is preferred. The polypeptides according to the invention may thus be expressed in genetically engineered cell lines eg. cell lines (such as Hela or BHK) transfected with, for example, a virus vector, and capable of constitutively expressing the FIV polypeptides according to the invention. Thus, for example, as typical of a suitable viral vector may be mentioned a recombinant vaccinia virus. As a convenient means of expression, a nucleic acid molecule according to this invention may be inserted into a plasmid vector downstream of a vaccinia virus promoter and flanked by vaccinia thymidine kinase (TK) sequences. The resultant recombinant vector is introduced into cells transfected with vaccinia virus. As a result of homologous recombination a TK recombinant vaccinia virus is generated which expresses the polypeptide.

Preferred expression systems according to the present invention include bacterial systems such as E. coli and Salmonella, yeast, vaccinia, baculoviruses, and mammalian cell systems (e.g. BHK cells).

Synthetic polypeptides according to the invention may also be prepared by chemical means, such as the well known Merrifield solid phase synthesis procedure.

The nucleic acid sequences and polypeptides according to the invention may be used to prepare vaccine compositions using methods well known in the art of vaccine manufacture.

Traditional vaccine formulations may comprise one or more polypeptides according to the invention together, where appropriate, with one or more suitable adjuvants eg. aluminium hydroxide, muramyl dipeptide, mineral or vegetable oils, novosomes, liposomes, saponins, Quil-A, Q5-21, Matrix pluronics or in the presence of one or more pharmaceutically acceptable carriers or diluents. Suitable carriers include liquid media such as saline solution appropriate for use as vehicles to introduce the polypeptides into a subject. Additional components such as preservatives may be included. Immune stimulating complexes (ISCOMS - Morein et al., Nature, 308: 457-460, 1984) have recently been found to be particularly effective as adjuvants in vaccine preparations.

Such vaccine compositions form a further aspect of the present invention, and the invention can thus be seen also to provide a composition for combatting FIV, preferably a vaccine composition for stimulating an immune response in an animal, preferably in a mammal, against FIV, said composition comprising one or more polypeptides comprising an amino acid sequence containing a part of the FIV envelope protein, said part consisting essentially of a sequence corresponding to the region of the FIV envelope protein spanning residues 483 to 567, or a portion thereof which is an FIV-combatting product or a functionally equivalent variant thereof, together with, optionally, one or more adjuvants, and a pharmaceutically acceptable carrier or diluent.

An alternative way of presenting the vaccine according to the invention, is to administer to the animal an expression vector, capable of expressing the polypeptides in question such that the immunogenic polypeptides are expressed in situ in the animal. Such "in situ" expression of the antigenic polypeptides has been found to present the immunogen to the immune system of the animal in a particularly favourable manner, and immune responses elicited in this manner are generally significantly improved over those generated using more conventional techniques.

A further aspect of the invention thus includes a composition for combatting FIV, preferably a vaccine composition for stimulating an immune response in an animal and against FIV, said composition comprising an expression vector, or host cell having inserted therein a nucleotide sequence according to this invention for stimulation of an immune response directed against polypeptides encoded by the inserted nucleotide sequence, together with one or more pharmaceutically acceptable carrier or diluents.

Expression vectors suitable for such use are known in the art and include in particular viral vectors, notably pox virus vectors. Vectors worthy of particular mention include the vaccinia virus, fowl pox virus, canary pox virus, and feline rhinotracheitis, feline calicivirus, feline panleukopenia, adenovirus and bacteria (eg. Salmonella) as described for example by Ada et al., Vaccine 8: 425-437, 1990. Advantageously a fusion protein may be expressed in such a system, where the heterologous protein part (the "carrier") comprises all or a portion of a transmembrane protein (eg. influenza virus HA). When expressed in eukaryotic cells infected with such a recombinant virus, the fusion protein is generally glycosylated and transported to the cell surface through which it protrudes thereby presenting the synthetic polypeptides (or their epitopes) on the outside of the cell surface.

Polypeptides expressed in such systems may include one or more shorter or longer length polypeptide sequences from the region between residues 483-567 of the FIV envelope protein.

Administration of a composition eg. a vaccine composition according to the invention may take place by any of the conventional routes, eg. orally or parenterally such as by intramuscular or intradermal, and especially subcutaneous injection, optionally at intervals eg. two injections at a 7-42 day interval. Typically a polypeptide is administered in an amount of 1 to 1000 µg per dose, more preferably 2 to 100 µg per dose.

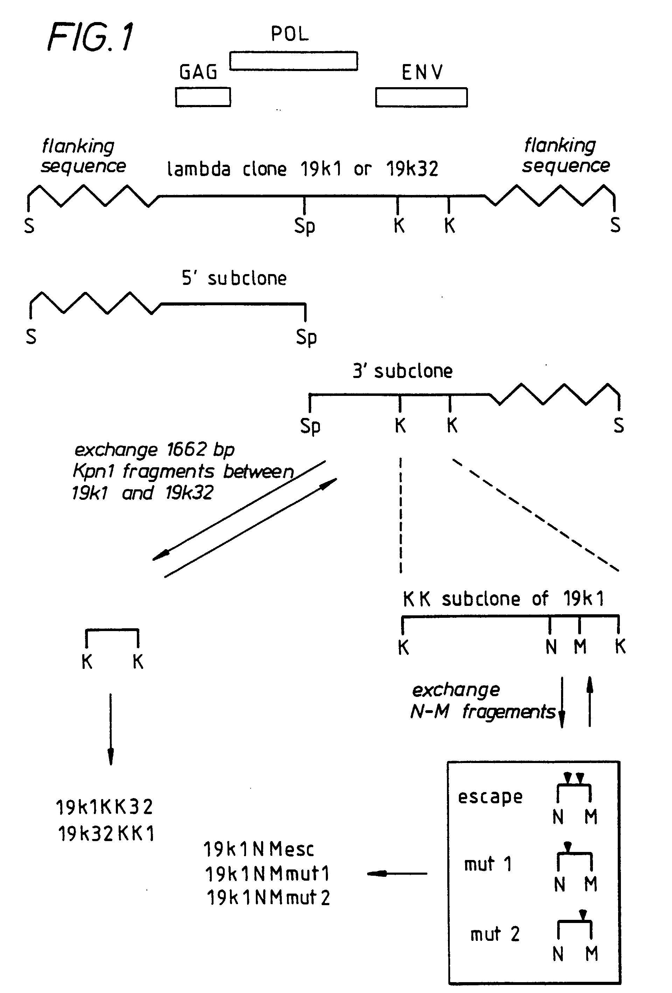

The invention will now be discussed in more detail in the following non-limiting Examples. In these Examples
Figure 1 shows a schematic representation of the construction of chimeric FIV clones. The uppermost bars represent the major open reading frames gag, pol and env. Restriction enzyme cleavage sites are indicated as follows: K, Kpn I; M, Mst II; N, Nsi I; S, Sal I; Sp, Sph I. Individual point mutations are indicated (▼);

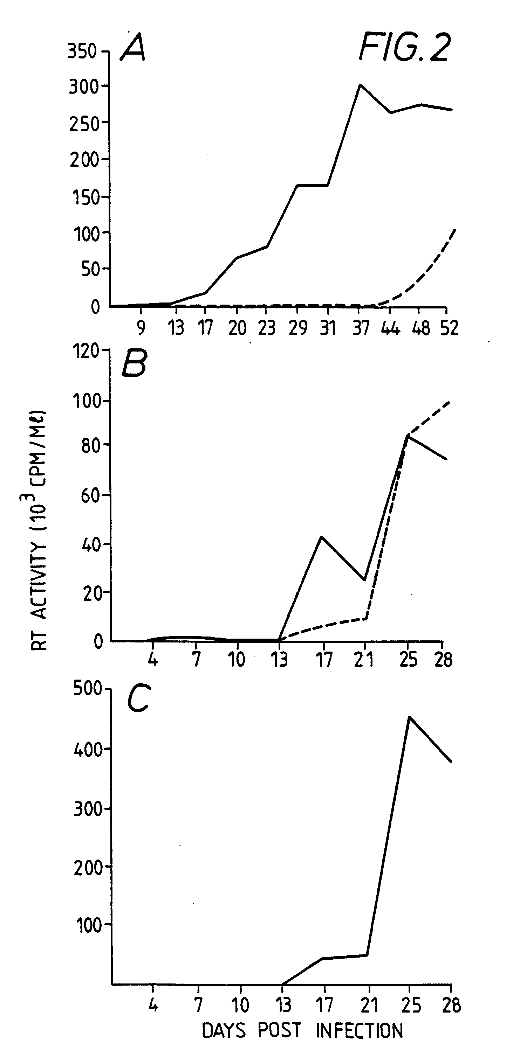

Figure 2 shows the kinetics of virus replication expressed as reverse transcriptase (RT) activity (cpm x 10³) demonstrated in the culture of escape mutants FIV 19K1 (A), FIV 19K1A (B), FIV 19K1B (C), in the presence of serum 5/4/22 (dashed line) or SPF control serum (solid line);

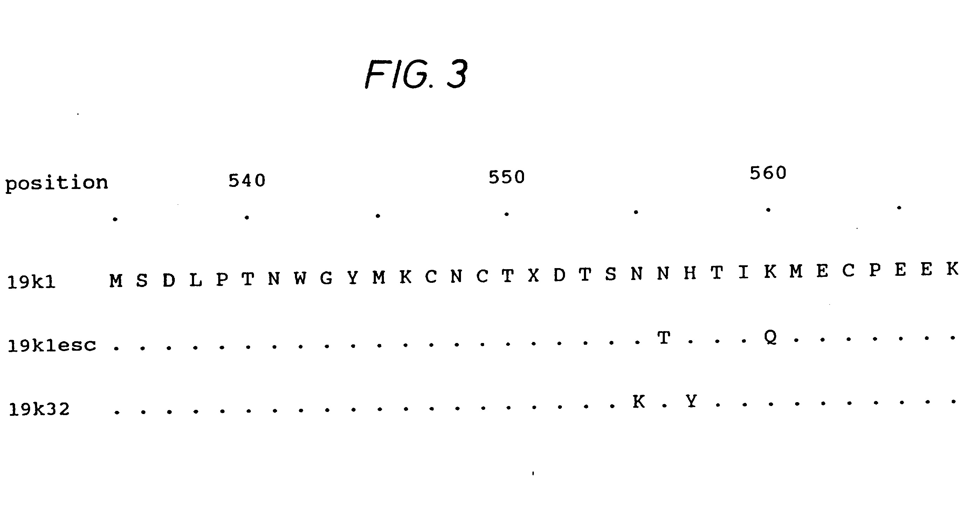

Figure 3 shows a comparison of the amino acid sequences of hypervariable region 5 of the envelope genes of the molecular clones 19K1, 19K32 and 19K1 esc; and

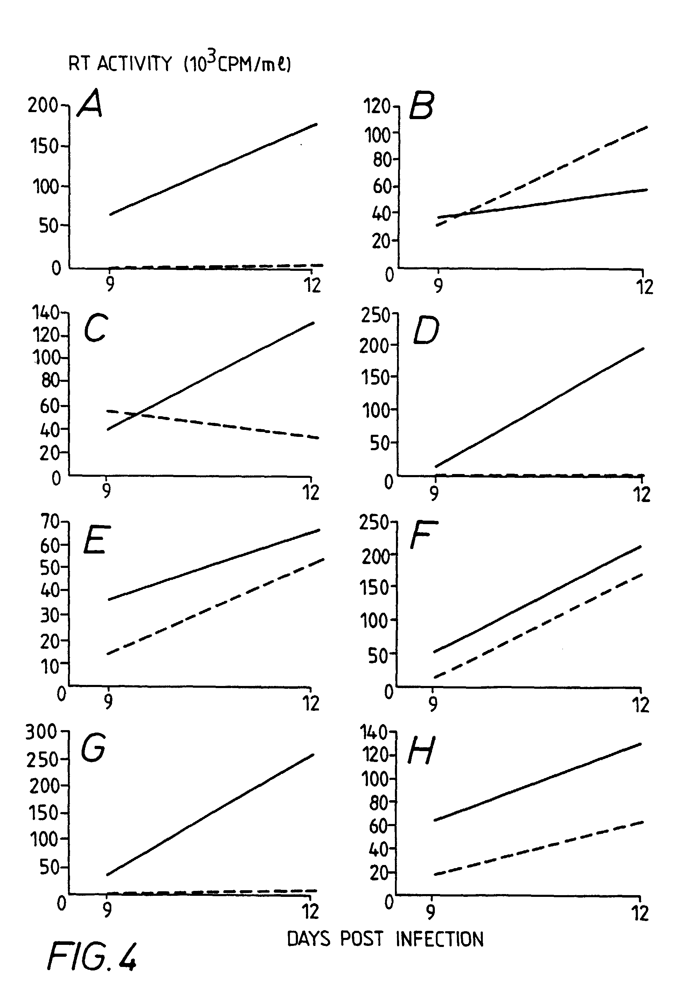

Figure 4 shows the results of neutralisation experiments using viral progency of parental and chimaeric FIV clones and 19K1 esc with serum S14/22 (dashed line) or SPF serum (solid line) as determined by measuring RT activity (cpm x 10³) at 9 to 12 days post-infection, (A) FIV 19K1; (B) FIV 19K32; (C) 19K1KK32; (D) 19K32KK1; (E) FIV19K32; (F) FIV 19K1N Mesc; (G) FIV 19K1 Mutl; (F) FIV 19K1 Mut2.

### EXAMPLE 1

### Material and Methods

**Virus.** Viral progeny of replication competent molecular clones 19kl and 19k32 and the biological isolate FIV A'dam 19 were obtained as described previously (Siebelink et al. 1992 supra). Briefly, molecular clones 19kl and 19k32 were directly isolated from the DNA from bone marrow cells of a naturally FIV infected cat designated A'dam 19. DNA of these molecular clones was transfected into Crandell feline kidney cells (CrFK). Virus production was continued by cocultivation of this culture with ConA and IL, stimulated peripheral blood mononuclear cells (PBMC) of an SPF cat. After 72 hours the PBMC were cultured separately in RPMI 1640 (Gibco), supplemented with penicillin (100 lU/ml), streptomycin (100 µg/ml). L-glutamine (2 mM), β-mercaptoethanol (2 x 10⁻⁵) and IL, (100 1U/ml) hereafter designated as culture medium (CM). When FIV antigen could be detected in these cultures the supernatant was filtered through a 200 nm filter and stored in aliquots at -135°C. The biological isolate FIV A'dam 19 was obtained by stimulating PBMC of cat A'dam 19 with 8 ConA for three days and cultured further in presence of 100 1U 1L/ml. Culture supernatant was filtered and stored in aliquots at 135°C when FIV antigen could be detected in the supernatant.

A 10 weeks old SPF cat (Cat No. 14) was infected with viral progeny of clone 19k1 (FIV 19k1) by intraperitoneal inoculation of 10² 50% tissue culture infectious doses. Virus was reisolated from this cat at 2 and 32 weeks post infection (p.i) as described above and designated FIV 19k1 R2 and FIV 19k1 R32 respectively.

Sera. Serum of a naturally FIV infected cat, A'daml9 was designated serum A19 (see Siebelink, 1992 supra). Serum of cat 14 was taken prior to infection (preserum) and at 22 weeks post infection (designated S14/22). Serum of a non-infected SPF cat was used as a control serum (designated S-SPF). All sera were heat inactivated for 1 hour at 56°C prior to use.

**Gag and Env-specific ELISA.** The presence of antibodies directed against the envelope protein of FIV was detected in a recombinant FIV envelope protein ELISA as follows: cell lysates of recombinant vaccinia virus (rVV) VGR657 expressing the env gene (G. Rimmelzwaan et al., The 1st International Conference on immunodeficiency virus researches held at U.C. Davis, September 4-7, 1991)(or wild type vaccinia virus) infected RK13, BHK or HeLa cells were coated onto ELISA plates (Costar RIA/EIA, high bonding) in 0.1M NaAc buffer. pH 5.5 in 50 µl volumes for 16 hours at room temperature. The wells were then emptied and fixed with 4% w/v paraformaldehyde for 10 minutes at room temperature. After washing the plates were blocked with PBS containing 0.05% Tween-80 and 10% FCS.

After coating and blocking of the microtiter plates, 50 µl of two fold serial dilutions of serum samples in PBS-TB supplemented with 5% NaCl were added at a starting dilution of 1:40 or 1:20. Then the plates were incubated for one hour at 37°C. Subsequently, 50 µl volumes of a biotin conjugated mouse MAb directed to cat IgG (Sigma, St Louis, USA) or an HRP conjugated goat anti-cat IgG antibody preparation (Cappel Cooper Biomedicals, Malvern, USA) were added and the plates were incubated for one hour at 37°. HRP bound streptavidin (Amersham International UK) was allowed to bind to biotin for 30 minutes at 37°C. After each of these incubations the plates were washed with DWT and subsequently developed with Tetramethylbenzidine (TMB) as follows: 100µl of sutstrate solution consisting of 0.1mg/ml TMB and 0.003% H₂O₂ in 0.1 M NaAc Buffer, pH5.5 were added to each well. After incubation for 10 minutes at 20°C, 100µl volumes of 2M H₂SO₄ were added to stop the colour reaction. OD₄₅₀ values were determined in a Titertek multiscan. Titres were given as the reciprocals of the dilutions still giving more than three times background values obtained with control antigen coated plates.

Antibodies raised against gag proteins were detected using a commercial available FIV p24/p17 antibody ELISA as recommended by the manufacturer (European Veterinary Laboratory, Amsterdam, The Netherlands).

**Reverse transcriptase assay.** Reverse transcriptase (RT) -activity was determined as previously described (Siebelink, 1992, supra).

**Virus neutralization assay.** Since low passage virus levels were used to avoid additional mutations by in vitro culturing, and the virus titer of the stocks grown on feline PBMC or thymocytes (Siebelink, supra) is low, (approximately 10²) the viral input was standardized by RT activity. Virus stocks of the biological isolate FIV A'dam 19, the molecular clones FIV 19kl and FIV 19k32 end the re-isolates FIV 19k1R2 and FIV 19k1R32 were tested in RT assay. For this assay, 450 µl of diluted virus stocks containing a RT activity of 5 x 10⁴ CPM/ml were incubated with 50 µl of a serum sample for 60 minutes at 37°C in a 24 wells plate (Costar).
Concanavalin A (ConA) - and interleukin-2 (IL-2) - stimulated PBMC or thymocytes (10⁶) were added. After 60 minutes at 37°C the cells were washed and incubated for 14 days in CM (RPMI 1640 [GIBCO], pencillin [100 IU/ml] streptomycin [100 µg/ml], L-glutamine [2mM], β-mercaptoethanol [2x10⁻⁵M], IL-2 [100 IU/ml]) supplemented with 2% of the respective serum sample. RT activity was determined as a measure for virus production. Virus neutralisation was considered positive when the RT activity did not increase over two times the background defined as the mean RT activity of uninfected cells. In previous experiments was observed that the RT activity of infected cultures was at least five times the background (not shown).

**Generation of escape mutants.** ConA and IL₂ stimulated PBMC of an SPF cat (10⁶) were infected with FIV 19k1, which is designated parent clone. After 24 hours the cultures were divided in two identical flasks. To one of the cultures 2% of serum S14/22 was added and to the other one 2% S-SPF (control serum). The cultures were harvested weekly, replaced with fresh CM supplemented with 2% of the respective serum and tested for the presence of RT activity. When RT activity over 5 times background values could be measured in the culture supernatant the supernatant was filtered through a 220 nm pore-size filter and stored at -135°C. Virus derived from these cultures are designated 19k1A and 19k1B for virus derived from the cultures in the presence of S14/22 and control serum respectively. Dilutions of the culture supernatants of 19k1A and 19k1B containing RT activity of 5 x 10⁴ cpm/ml were incubated with 10% of serum sample S14/22 or control serum for 60 minutes at 37°C. ConA and IL-2 stimulated PBMC of an SPF cat (10⁶) were added to each culture. After 60 minutes incubation at 37°C, the cells were washed and cultured in CM supplemented with 2% of the respective serum. The culture supernatant was tested for presence of RT activity twice a week. When RT activity could be demonstrated the culture supernatant was filtered through a 220 nm pore-size filter and stored at -135°C. The viruses were designated 19k1A.A and 19k1A.B for 19k1A propagated in presence of S14/22 or control serum (S-SPF) respectively and 19k1B.A and 19k1B.B for 19k1B propagated in presence of S14/22 and control serum respectively. 19k1A.A was further designated 19k1esc.

**Sequence analysis.** The sequence of the envelope gene of the parental clone 19k1 is described previously (Siebelink, 1992 supra). The envelope gene of the escape mutant 19k1esc was amplified by PCR. PBMC (10⁵) from the culture 19k1esc were pelleted and lysed with 100 µl K-buffer (50 mM KCl, 10 mM Tris pH 8.4, 1.5 mM MgCl₂ 0.5% Tween-20 and 100 µg/ml proteinase K) for 45 minutes at 56°C. The reaction was stopped by incubating the mixture for 5 minutes at 95°C. Two oligo nucleotide primers were designed (primer 1, 5'-GGCGAATTCATGGCAGAAGGATTTGTAGCC - 3' and primer 2, 5'-TATGCATGCCATTCCTCCTCTTTTTCAGACATGCC - 3') which contained an EcoRl and Sphl restriction enzyme cleavage site (underlined) respectively to facilitate subsequent cloning into pUC19. PCR reactions were carried out in a separate laboratory to avoid contamination. In a volume of 100 µl containing 10 µl of the cell lysate, 50 mM KCl, 10 Mm Tris pH 8.4. 1.5 mM MgCl₂, 0.02% gelatine, 250 µM deoxynucleotide-triphosphates, 1 µM of each primer and 2.5 units of Taq polymerase. Samples were overlaid with 100 µl of mineral oil to avoid evaporation and the subjected to 35 amplification cycles consisting of a denaturating step (94°C, 1 min.) a primer-annealing step (60°C, 1 min.) and a primer-extension step (72°C, 2 min.). Negative controls (non-infected PBMC from an SPF cat) were amplified in parallel. Amplification products were analysed by agarose gel electrophoresis (0.8%) for presence of the appropriately sized DNA fragments. The excess of primers in the PCR reaction was eliminated using Centricon-30 microconcentrator (Amicon). DNA was digested with EcoRl and Sphl and cloned into pUC19. The subclones were used for the dideoxy nucleotide chain termination reaction (Sanger F. 1981. Determination of nucleotide sequences in DNA. Science 214: 1205-1210) of the envelope gene. The nucleotide and protein alignments were done with Lasergene software (DNAstar inc. London, UK). The observed nucleotide variation of 19klesc was confirmed by using an independent PCR amplification to exclude Tag errors.

**Generation of chimeric clones.** The construction of chimeric clones is schematically represented in Figure 1. Restriction mapping identified a common and unique internal Sphl site in both FIV genomes. To generate recombinant viruses the lambda clones 19k1 and 19k32 were digested with SalI and SphI. The SalI and SpbI fragments generated after digestion were cloned into pUC19 plasmid vector of which the KpnI site had previously been deleted. The plasmids containing the 5' long terminal repeat (LTR), gag and 5' part of Pol were designated 5' subclones. Plasmids containing the 3' part of Pol, env and 3' LTR were designated 3' subclones and were used to exchange internal 1666 base pair (bp) KpnI fragments between the clones 19k1 and 19k32.

The 1662 bp KpnI fragments of clone 19kl were also cloned into pUC19 and was designated 3'KK subclone of 19k1. A 144 bp NslI - Mst2 fragment of the 19k1esc envelope (see sequence analysis of this section) was exchanged as was a 144-bp NslI - Mst II fragment of clone 19k1 in which one nucleotide was substituted by site directed mutagenesis at either position 1667 or position 1678. Site directed mutagenesis was performed by PCR on the 3' subclone of 19k1. To that end three oligonucleotides were designed: Primer I, 5-CCTTATTATGCATTTCAATATGACAAAAGCTG-3' contained an NslI site (underlined); primer mutl, 5-GCCTTTTTCCTCAGGACATTCCATTTTTATTGTGTGAGTATTG-3' contained an Mst2 site (underlined) and a T-to-G substitution at position 1667 (bold) and primer mut2, 5'-GCCTTTTTCCTCAGGACATTCCATTTGTATTG-3' contained a Mst2 site (underlined) an a T-to-G substitution at position 1678 (bold). Two PCR reactions were carried out using in both reactions primer 1 to prime from the 5' end. In one reaction primer mutl was used while in the other reaction primer mut2 was used to prime from the 3' end. The conditions for the PCR reaction are described in sequence analysis of this section. PCR fragments were digested with NslI and Mst2 and cloned into the 3' KK subclone of 19kl. The mutagenesis was confirmed by sequence analysis.

The 1662 bp KpnI fragments from the 3' KK subclones in which 144 bp NslI and Mst2 fragments were exchanged were cloned into the 3' subclone of 19k1. 25 µg of each of the 3' subclones and the 5' subclones were mixed, digested with SphI and ligated. After digestion with SalI the constructs were transfected into Crandell feline kidney cells (CrFK) as previously described (Siebelink, 1992 supra). Virus production was rescued by cocultivation of transfected cells with ConA and IL, stimulated PBMC for three days. Cultures were monitored for syncytium formation of PBMC and RT activity in the culture supernatant. When RT activity could be measured culture supernatant was filtered through an 220 nm filter and stored in aliquots at -135°C.

### Results

**Infection of cats.** Upon experimental infection with viral progeny of clone 19k1 cat 14 seroconverted within 4 weeks. The gag- and Env-specific serum antibodies measured by ELISA reached plateau levels within 8 weeks after infection (Table 1). Virus was re-isolated 2 and 32 weeks post infection (p.i.) (Table 1). The serum collected 22 weeks p.i. (serum S14/22) and the two virus isolatates (FIV 19k1R2 and FIV 19k1R32, respectively) from this cat were used in the experiments described in this Example.

**Table 1.**

| Anti-FIV serum antibody response of cat 14 determined by Gag- and Env-specific ELISA and virus isolation after infection with FIV 19k1 of cat 14. | | | |
|---|---|---|---|
| | antibody response | | |
| weeks p.i. | (p24/p17) gag | (gp120) env (reciprocal titre) | virus isolation |
| 0 | <50 | <40 | ND |
| 2 | <50 | 40 | + |
| 4 | 722 | 640 | ND |
| 6 | 902 | >5120 | ND |
| 6 | 1031 | >5120 | ND |
| 11 | 1186 | >5120 | ND |
| 22 | 1079 | >5120 | ND |
| 32 | 1036 | >5120 | + |
| OD₄₅₀ (x 10⁻³), (optical Density at 450nm) ND: not determined. | | | |

**Virus neutralization.** Serum from cat 14 obtained 22 weeks p.i. (S14/22) was tested for presence of neutralizing antibodies against viral progeny of molecular clones FIV 19k1 and FIV 19k32 and the biological isolates FIV A'dam 19, FIV 19k1R2 and FIV 19k1R32. Serum of an SPF cat was used as control. The results are listed in Table 2.

**Table 2.**

| Virus neutralisation of an FIV isolate, molecular clones, and FIV re-isolates with serum S14.22 and SPF serum | | |
|---|---|---|
| | RT activity (cpm/ml) after neutralisation with: | |
| Virus | S14/22 serum | SPF serum |
| FIV A' dam19 | 24219 | 61480 |
| 19k19 | 157 | 12333 |
| 19k32 | 26864 | 59187 |
| FIV 19k1R2 | 82 | 58442 |
| FIV 19k1R32 | 103 | 18145 |

Virus growth as determined by RT activity could be demonstrated in all cultures in the presence of the control serum. Replication of virus could also be detected in the presence of S14/22 in the cultures of the biological isolate FIV A'dam 19 and FIV 19k32. However, no virus replication could be detected in the culture infected with FIV 19k1 and the re-isolates FIV 19k1R2 and FIV 19k1R32 cultured in presence of S14/22, indicating complete neutralization. Serum S14/22 which was absorbed with protein-A Sepharose beads to remove antibodies IgG (particularly failed to neutralise molecular clone 19k1 which shows that serum immunoglobulin is responsible for the observed neutralisating activity (not shown).

**Generation of escape mutants.** FIV 19k1-infected PBMC from an SPF cat were cultured in presence of S14/22 (culture A, designated 19k1A) or control serum (culture B, designated 19k1B). Within 17 days RT activity could be measured in the supernatant of the culture 19k1B. After 44 days of culture RT activity could also be detected in culture 19k1.A (Fig. 2A). The RT activity increases during time of culture. No RT activity could be demonstrated in the non-infected culture which were cultured in presence of S14/22 or control serum for 52 days of culture (data not shown).

The supernatant of the cultures 19k1A and 19k1B were collected and filtered 52 days after infection. The culture supernatants were incubated with serum S14/22 or control serum. ConA and IL-2 stimulated PBMC from an SPF cat were added to the virus - serum mixture and cultured further in the presence of 2% of the respective serum, S14/22 or control serum. The cultures were designated 19k1A.A and 19k1A.B for the cultures of 19k1A cultured in presence of S14/22 or control serum respectively (Figure 2B) and 19k1B.A and 19k1B.B for the cultures of 19k1B cultured in the presence of S14/22 or control serum respectively (Figure 2C). RT activity could be demonstrated after 17 days of infection in both cultures of 19k1A. The kinetics of virus replication of the 19k1A.A were similar to that of the control 19k1A.B (Figure 2B) and 19k1B.B (Figure 2C). As expected no virus replication was observed in the culture 19k1B.A (Figure 2C). These results show that 19k1A.A is resistant to virus neutralisation by serum S14/22.

**Sequence analysis.** Cells of the culture 19k1A.A further designated 19k1esc were lysed and the envelope gene of the FIV genome, integrated in the cellular DNA, was amplified by PCR. The PCR product was digested with EcoRl and Sph1 and cloned into PUC19. The envelope gene of 19k1esc was sequenced and compared with the envelope sequence of the parental clone 19k1 and clone 19k32 (Fig 3). At position 468 in the hyper-variable region 2 (HV-2) a silent transition of T-to-C was observed (not shown). Two A-to-C transversions were observed in HV-5. One at position 1667 causes an amino acid substitution of Asn to Thr (Amino acid position 556) whereas the other at position 1678 causes an amino acid substitution of Lys to Gln (Amino acid position 560). Comparison of the nucleotide sequence of the envelope gene of clone 19kl with 19k32, which exhibited the same phenotype as 19k1esc in an neutralization assay using S14/22, showed the presence of a T-to-G transversion at position 1665 and a transition of C-to-T at position 1669 resulting in a substitution of Asn with Lys (Amino acid position 555) and His with Tyr (amino acid position 557) respectively (Figure 3).

**Virus neutralisation of chimeric clones.** Viral progeny of the parental clone 19kl and those in which the envelope gene was partially exchanged or an amino acid was substituted by site directed mutagenesis and the escape mutant 19k1esc were incubated with serum S14/22 or SPF control serum prior to infection of ConA and IL-2 stimulated thymocytes. The results of this experiment are similar to those of a number of previous experiments in which the chimeric clones were tested separately for neutralisation by serum S14/22. RT activity was measured 9, 12 and 15 days post infection. RT activity could be measured in all the control cultures 9 days post infection and continued to increase through 12 days post infection (Figure 4). As expected FIV 19k1 was neutralised by serum S14/22 and not by the SPF control serum (virus growth was demonstrated in the control culture) (Fig. 4A). As before, FIV 19k32 was not neutralised by S14/22 (Fig. 4B). To investigate whether the difference in virus neutralisation between the two molecular clones was caused by the differences in the surface protein, we exchanged the 1662 bp KpnI fragment. The chimeric clones were designated 19k1KK32 (19k1 containing the KpnI fragment of 19k32) and 19k32KK1 (19k32 containing the KpnI fragment 19k1). Chimeric clone 19k1KK32 was not neutralised (Fig. 4C) whereas chimeric clone 19k32KK1 was neutralised (Fig. 4D) with serum S14/22 indicating that an epitope within the 1662 KpnI fragment of the envelope gene is involved in neutralisation. In this experiment the RT activity in the supernatant of culture 19k1KK32 grown in presence of S14/22 tested 12 days post infection was low. In previous experiments an increase of RT activity was demonstrated from 9 to 12 days post infection.

The escape mutant of FIV 19k1 grown in presence of serum S14/22 was not neutralised by this serum (Fig. 4E). Comparison of the amino acid sequence of the envelope protein of 19kl and the sequence of the escape mutant showed two amino acid substitutions within the hypervariable region 5 (HV-5). To see whether these substitutions underlie the escape of FIV 19k1 from serum S14/22 we exchanged the 144 bp NslI - Mst2 fragment, containing the whole HV-5, of clone 19kl with that of the escape mutant. This chimera, designated 19k1NMesc was not neutralised by S14/22 (Fig. 4F) indicating that one or both amino acid substitutions are involved in the escape mechanism. By site directed mutagenesis the two amino acids were substituted separately. These point mutated clones were designated 19k1mut1 (position 556: Asn to Thr) and 19k1mut2 (position 560: Lys to Gln). FIV 19k1mut1 was neutralised by serum S14/22 (Fig. 4G) whereas FIV 19k1mut2 could not be neutralised by S14/22 (Fig. 4H) indicating that the point mutation at amino acid position 560 resulting in an amino acid substitution from Lys to Gln is responsible for escape of FIV 19k1 from serum S14/22. To investigate whether the differences in HV-5 between clones 19k1 and 19k32 also underlie also the differences in neutralisation with serum S14/22 the 144 bp NslI - MSt2 fragment was reciprocally exchanged between the two clones. This exchange, however, did not result in a change in virus neutralisation (not shown) indicating that another epitope might be involved.

### Summary

Serum from an SPF cat (No. 14) which was infected with viral progeny of molecular clone FIV 19kl obtained 22 weeks post infection could neutralize this virus in vitro. This serum could also neutralize virus which was re-isolated from this cat 2 and 32 weeks post infection. FIV 19k32 and the biological isolate FIV A'daml9 which were both isolated from the same cat as 19kl were not neutralized by this serum in vitro. The fact that FIV 19k32 which differs from FIV 19k1 only in 6 amino acids in the envelope protein is not neutralised suggests that the neutralizing antibodies in the serum S14/22 are directed to an epitope which is strictly type specific. In humans type specific HIV-1 neutralizing antibodies arise early after infection with HIV-1 and appear to react mainly to the so-called principal neutralising domain, the V3 - loop (Goudsmidt PNAS 85:4478-4482, 1988; Matsushita J.Virol 62:2107-2114. Palker, PNAS 85:1932-1936, 1988; Rusche, PNAS 85:3198-3202, 1988). Later in course of infection, group specific virus neutralizing antibodies are detected (Kang PNAS 88:6171-6175).

The fact that serum S14/22 which is obtained from an SPF cat which is infected with FIV 19k1 could only neutralise FIV 19k1 and re-isolates from cat 14 but not FIV 19k32 indicate that in cats following FIV infection type specific virus neutralising antibodies appear early after infection. Others have also shown that virus neutralising antibodies in cats could be detected within four weeks after experimental infection. (Fevereiro et al., J. Gen Virol 72: 617-622, 1991; Tozzini et al., J. Virol. Meth., 37: 241-251, 1992). Serum samples from cats either experimentally or naturally infected with different isolates of FIV could all neutralise the FIV Petaluma strain indicating that group specific virus neutralising antibodies were present. These virus neutralisation assays rely on a strain of FIV adapted to Crandel feline kidney (CrFK) cells. Most FIV isolates will not infect CrFK cells and adaptation of FIV to CrFK cells is necessary to propagate the virus in these cells. Differences in the target cells used could therefore well underlie the observed differences between the group specific neutralisation of FIV reported by others (Fevereiro, Tozzini supra) and the type specific neutralisation reported in this example. To determine the region in the envelope protein which is involved in virus neutralisation we generated escape mutants of FIV by propagating FIV19kl in the presence of serum from an SPF cat, obtained 22 weeks after infection with FIV 19k1. Comparison of the amino acid sequence of the envelope gene of the parental clone 19k1 and that of the escape mutant 19k1esc showed that two amino acids in the hyper-variable region 5 (HV-5) were mutated: an amino acid substitution of Asn to Thr at position 556 and of Lys to Gln at position 560. Comparison of the amino acid sequence of the parental clone 19k1 with clone 19k32, which is not neutralised by S14/22 showed also differences in HV-5: an amino acid substitution of Asn to Lys at position 555 and at position 557 of His to Tyr.

Single amino acid substitution in the transmembrane envelope protein of HIV-1 can cause resistance to neutralisation. (Reitz et al., Cell 54:57-63, 1988). However, the reported substitution of amino acid Ala to Thr at position 582 does not involve the neutralisation epitope (Wilson J. Virol 64: 3240-3248, 1990) which indicates that escape from neutralisation can arise through mutations outside the neutralising epitope. Changing of an amino acid outside of a neutralising epitope could e.g. change the structure of the envelope protein which can result in masking of a previously exposed neutralising epitope. In order to show that the amino acid substitution in the envelope gene of the escape mutant 19k1esc of FIV is the only cause for resistance to neutralisation with serum S14/22 site directed mutagenesis and partial gene exchangement was performed which led to the conclusion that a single mutation in HV-5 of the surface protein of FIV is sufficient to escape from the pressure exerted by neutralising antibodies.

Reciprocal exchange of a large fragment of the envelope gene of clone 19k1 and 19k32 showed that the neutralisation epitope or 19k1 for S14/22 is situated on the surface protein. Similarly exchange of the 144 bp NslI - Mst2 fragment containing the HV-5, between the clone 19kl and the escape mutant resulted in an exchange of neutralisation phenotype and substitution of a single amino acid in HV-5 resulted in escape from the neutralising antibodies in serum S14/22. However, the reciprocal exchange of the 144 bp NslI - Mst2 fragment between the clones 191 and 19k32 did not result in an exchange in neutralisation phenotype which demonstrates that functional differences between 19k1 and 19k32 with regard to neutralisation lie outside HV-5.

This study demonstrates that a single point mutation resulting in a substitution of Lys to Gln at amino acid position 560 underlies the escape from the neutralising antibodies in vitro.

### Example 2

An SPF cat was infected with viral progeny of molecular clone 19k1 as described in Example 1. Serum from this cat, obtained 22 weeks post infection, could neutralize viral progeny of this molecular clone but not that of 19k32 which is 99.3% identical to 19k1 in the envelop gene. By gene exchange and point mutations (see Example 1) we constructed chimeric clones and tested these in our virus neutralisation assay based on infection of feline thymocytes (see Example 1). It was shown that the exchange of a 1662 bp KpnI fragment, which contains 5 out of 6 substitution mutations, hanged the virus neutralizing phenotype. We then reciprocally exchanged a 144 bp NsiI. MstII fragment which contains 2 out of 6 substitution mutations in the hypervariable region 5. Exchange of this fragment did not change the phenotype (Table 3). The other three substitution mutations, a R-S at position 222, a N-D at position 454 and a L-S at position 483 were substituted by site directed mutagenesis. Only the leucine to serine substitution at position 483 in 19kl confered resistance to virus neutralisation (Table 3).

**Table 3:**

| Virus neutralisation of FIV molecular clones and chimeric clones | | |
|---|---|---|
| Virus | RT activity after neutralisation with: serum cat 14 | |
| | pre | week 22 p.1. |
| 19k1 | 55215 | 1296 |
| 19k1 NM32 | 291690 | 3558 |
| 19k1 222 | 690036 | 6774 |
| 19k1 454 | 586896 | 6561 |
| 19k1 483 | 622740 | 545613 |
| 19k32 | 465969 | 2506722 |
| 19k32 NM1 | 573012 | 429117 |
| 19k32 222 | 325815 | 586269 |
| 19k32 454 | 498948 | 331725 |
| 19k32 483 | 870699 | 613587 |

Through this mutation an additional potential glycosylation site (N.S) is introduced. Glycosylation of this site could mask a viral neutralising epitope, or could change the conformation of the protein through which virus-neutralising (VN), antibodies cannot recognise a neutralising epitope.

## Claims

1. A nucleic acid molecule, the nucleotide sequence of which corresponds to the region of the FIV env gene encoding residues 483 to 567 of the FIV envelope protein or a portion thereof encoding an FIV-combatting product or a sequence which is degenerate, has greater than 60% sequence homology therewith for use in the preparation of a composition for combatting FIV.

2. A nucleic acid molecule comprising a first nucleotide sequence consisting of a sequence corresponding to the region of the FIV env gene encoding residues 483 to 567 of the FIV envelope protein or a portion thereof encoding an FIV-combatting product or a sequence which has greater than 60% sequence homology therewith, together with at least one additional nucleotide sequence flanking said first nucleotide sequence, the additional nucleotide sequence comprising no more than 150 bases.

3. A nucleic acid molecule as claimed in claim 1 or claim 2 wherein said nucleotide sequence encodes an FIV-combatting product and (i) includes codons of the FIV env gene encoding residues selected from residue 483 and residues 549 to 567 of the FIV envelope protein in discontinuous arrangement; or (ii) includes the codon of the FIV env gene encoding residue 483 of the FIV envelope protein; or (iii) consists of the sequence of the FIV env gene encoding residues 549 to 567 of the FIV envelope protein.

4. Use of a nucleotide sequence corresponding to the region of the FIV env gene encoding residues 483 to 567 of the FIV envelope protein or a portion thereof encoding an FIV-combatting product or a sequence which is degenerate, has greater than 60% sequence homology therewith, in the preparation of a composition for combatting FIV.

5. Use of a nucleic acid molecule comprising a nucleotide sequence encoding a polypeptide capable of raising neutralising antibodies against FIV, consisting essentially of one or more antigenic determinant-encoding regions from the regions of the FIV env gene encoding residues 483-567 of the envelope protein for the preparation of a composition for combatting FIV.

6. A synthetic polypeptide consisting essentially of an amino acid sequence corresponding to the region of the FIV envelope protein spanning residues 483 to 567 or a portion thereof which is an FIV-combatting product, or a functionally equivalent variant thereof.

7. A synthetic polypeptide as claimed in claim 6 for use in combatting FIV.

8. A synthetic polypeptide as claimed in claim 6 or claim 7, in the form of a fusion protein comprising an additional polypeptide fused to said amino acid sequence, or which is coupled to a carrier protein or polypeptide.

9. Use of a polypeptide corresponding to all or a portion of the region of the FIV envelope protein spanning residues 483 to 567, or a functionally equivalent variant thereof, for the preparation of a composition for combatting FIV.

10. An expression or cloning vector comprising a nucleic acid molecule as defined in any one of claims 1 to 5.

11. A host cell containing a nucleic acid molecule as defined in any one of claims 1 to 5.

12. A method for preparing a synthetic polypeptide as defined in either one of claims 7 and 8 which comprises culturing a host cell containing a nucleic acid molecule as defined in any one of claims 1 to 5, under conditions whereby said polypeptide is expressed, and recovering said polypeptide thus produced.

13. A composition for combatting FIV comprising one or more polypeptides corresponding to the region of the FIV envelope protein spanning residues 483 to 567, or a portion thereof which is an FIV-combatting product, or a functionally equivalent variant thereof, or an expression vector, or host cell having inserted therein a nucleotide sequence as defined in any one of claims 1 to 5, for stimulation of an immune response directed against polypeptides encoded by the inserted nucleotide sequence, together with, optionally, one or more adjuvants, and a pharmaceutically acceptable carrier or diluent.

## Patentansprüche

1. Nucleinsäuremolekül, dessen Nucleinsäuresequenz der Region des FIV **env**-Gens entspricht, welche die Reste 483 bis 567 des FIV-Hüllproteins codiert, oder eines Teils davon, welcher ein FIV-bekämpfendes Produkt codiert, oder einer Sequenz, die degeneriert ist, eine 60 % größere Sequenzhomologie damit hat, zur Verwendung bei der Herstellung einer Zusammensetzung zur Bekämpfung von FIV.

2. Nucleinsäuremolekül, umfassend eine erste Nucleotidsequenz, bestehend aus einer Sequenz entsprechend der Region des FIV **env**-Gens, welches die Reste 483 bis 567 des FIV-Hüllproteins codiert, oder eines Teils davon, welcher ein FIV-bekämpfendes Produkt codiert, oder einer Sequenz, die eine Sequenzhomologie von größer als 60 % damit hat, zusammen mit mindestens einer zusätzlichen Nucleotidsequenz, die die erste Nucleotidsequenz flankiert, wobei die zusätzliche Nucleotidsequenz nicht mehr als 150 Basen umfaßt.

3. Nucleinsäuremolekül nach Anspruch 1 oder 2, worin die Nucleotidsequenz ein FIV-bekämpfendes Produkt codiert, und (i) Codons des FIV **env**- Gens umfaßt, welches Reste, ausgewählt aus den Resten 483 und den Resten 549 bis 567 des FIV-Hüllproteins in nicht zusammenhängender Anordnung codiert; oder (ii) das Codon des FIV **env**-Gens umfaßt, welches den Rest 483 des FIV-Hüllproteins codiert; oder (iii) aus der Sequenz des FIV **env**-Gens besteht, welche die Reste 549 bis 567 des FIV-Hüllproteins codiert.

4. Verwendung einer Nucleotidsequenz entsprechend der Region des FIV **env**-Gens, codierend die Reste 483 bis 567 des FIV-Hüllproteins oder eines Teils davon, welches ein FIV-bekämpfendes Produkt codiert, oder eine Sequenz, die degeneriert ist und eine Sequenzhomologie von größer als 60 % davon hat, zur Herstellung einer Zusammensetzung zur Bekämpfung von FIV.

5. Verwendung eines Nucleinsäuremoleküls, umfassend eine Nucleotidsequenz, codierend ein Polypeptid, das zur Erzeugung von neutralisierenden Antikörpern gegen FIV in der Lage ist, das im wesentlichen aus einer oder mehreren antigen determinant-codierenden Regionen aus den Regionen des FIV **env**-Gens, codierend die Reste 483 bis 567 des Hüllproteins umfaßt, zur die Herstellung einer Zusammensetzung zur Bekämpfung von FIV.

6. Synthetisches Polypeptid, bestehend im wesentlichen aus einer Aminosäuresequenz, entsprechend der Region des FIV-Hüllproteins, welches die Reste 483 bis 567 kodiert, oder eines Teils davon, welcher ein FIV-bekämpfendes Produkt ist, umfaßt, oder eine funktionale, äquivalente Variante davon.

7. Synthetisches Polypeptid nach Anspruch 6 zur Verwendung zur Bekämpfung von FIV.

8. Synthetisches Polypeptid nach Anspruch 6 oder 7 in Form eines Fusionsproteins, umfassend ein zusätzliches Polypeptid, das an die Aminosäuresequenz fusioniert ist, oder das an ein Trägerprotein oder Polypeptid gekoppelt ist.

9. Verwendung eines Polypeptids entsprechend der gesamten oder eines Teils der Region des FIV-Hüllproteins, welche die Reste 483 bis 567 umspannt, oder eine funktionale, äquivalente Variante davon zur Herstellung einer Zusammensetzung zur Bekämpfung von FIV.

10. Expressions- oder Clonierungsvektor, umfassend ein Nucleinsäuremolekül, definiert nach einem der Ansprüche 1 bis 5.

11. Wirtszelle, enthaltend ein Nucleinsäuremolekül, definiert nach einem der Ansprüche 1 bis 5.

12. Verfahren zur Herstellung eines synthetischen Polypeptids, definiert entweder nach einem der Ansprüche 7 und 8, welches die Kultur einer Wirtszelle umfaßt, enthaltend ein Nucleinsäuremolekül, definiert nach einem der Ansprüche 1 bis 5, unter Bedingungen, unter denen das Polypeptid exprimiert wird, und Gewinnen des so produzierten Polypeptids.

13. Zusammensetzung zur Bekämpfung von FIV, umfassend ein oder mehrere Polypeptide, entsprechend der Region des FIV-Hüllproteins, welches die Reste 483 bis 567 umspannt, oder eines Teils davon, welcher ein FIV-bekämpfendes Produkt ist, oder eine funktional äquivalente Variante davon, oder ein Expressionsvektor oder Wirtszelle mit einer darin eingefügten Nucleotidsequenz, definiert nach einem der Ansprüche 1 bis 5, zur Stimulierung einer Immunantwort, gerichtet gegen Polypeptide, die durch eingefügte Nucleotidsequenz codiert werden, zusammen mit wahlweise einem oder mehreren Adjuvanzien, und einem pharmazeutisch verträglichen Träger oder Verdünner.

## Revendications

1. Molécule d'acide nucléique, dont la séquence nucléotidique correspond à la région du gène env de FIV codant les résidus 483 à 567 de la protéine d'enveloppe de FIV ou à une portion de celle-ci codant un produit luttant contre le FIV ou à une séquence qui est dégénérée, qui a avec celle-ci une homologie séquentielle supérieure à 60% pour l'utilisation dans la préparation d'une composition pour combattre FIV.

2. Molécule d'acide nucléique comprenant une première séquence nucléotidique constituée d'une séquence correspondant à la région du gène env de FIV codant les résidus 483 à 567 de la protéine d'enveloppe de FIV ou à une portion de celle-ci codant un produit luttant contre le FIV ou à une séquence qui a avec celle-ci une homologie séquentielle supérieure à 60%, avec au moins une séquence nucléotidique supplémentaire adjacente à ladite première séquence nucléotidique, la séquence nucléotidique supplémentaire ne comprenant pas plus de 150 bases.

3. Molécule d'acide nucléique selon la revendication 1 ou la revendication 2 dans laquelle ladite séquence nucléotidique code un produit luttant contre le FIV et (i) comprend des codons du gène env de FIV codant les résidus sélectionnés dans le groupe constitué par le résidu 483 et les résidus 549 à 567 de la protéine d'enveloppe de FIV suivant un arrangement discontinu ; ou (ii) comprend le codon du gène env de FIV codant le résidu 483 de la protéine d'enveloppe de FIV ; ou (iii) est constitué de la séquence du gène env de FIV codant les résidus 549 à 567 de la protéine d'enveloppe de FIV.

4. Utilisation d'une séquence nucléotidique correspondant à la région du gène env de FIV codant les résidus 483 à 567 de la protéine d'enveloppe de FIV ou à une portion de celle-ci codant un produit luttant contre le FIV ou à une séquence qui est dégénérée, qui a avec celle-ci une homologie séquentielle supérieure à 60%, dans la préparation d'une composition pour combattre FIV.

5. Utilisation d'une molécule d'acide nucléique comprenant une séquence nucléotidique codant un polypeptide apte à dresser des anticorps neutralisants contre FIV, constituée essentiellement d'une ou plusieurs régions codant le déterminant antigénique provenant des régions du gène env de FIV codant les résidus 483 à 567 de la protéine d'enveloppe pour la préparation d'une composition pour combattre FIV.

6. Polypeptide de synthèse constitué essentiellement d'une séquence aminoacide correspondant à la région de la protéine d'enveloppe de FIV s'étendant du résidu 483 au résidu 567 ou à une portion de celle-ci qui est un produit luttant contre le FIV, ou à un variant de celle-ci fonctionnellement équivalent.

7. Polypeptide de synthèse selon la revendication 6 pour l'utilisation dans la lutte contre le FIV.

8. Polypeptide de synthèse selon la revendication 6 ou la revendication 7, ayant la forme d'une protéine de fusion comprenant un polypeptide additionnel ayant fusionné avec ladite séquence aminoacide, ou qui est couplé à un porteur protéinique ou polypeptidique.

9. Utilisation d'un polypeptide correspondant à tout ou partie de la région de la protéine d'enveloppe de FIV s'étendant du résidu 483 au résidu 567, ou à un variant de celle-ci fonctionnellement équivalent, pour la préparation d'une composition pour lutter contre FIV.

10. Vecteur d'expression ou de clonage comprenant une molécule d'acide nucléique selon l'une quelconque des revendications 1 à 5.

11. Cellule hôte contenant une molécule d'acide nucléique selon l'une quelconque des revendications 1 à 5.

12. Procédé de préparation d'un polypeptide de synthèse selon la revendication 7 ou la revendication 8 qui comprend la culture d'une cellule hôte contenant une molécule d'acide nucléique selon l'une quelconque des revendications 1 à 5, dans des conditions dans lesquelles ledit polypeptide est exprimé, et la collecte dudit polypeptide ainsi produit.

13. Composition pour lutter contre le FIV comprenant un ou plusieurs polypeptides correspondant à la région de la protéine d'enveloppe de FIV s'étendant du résidu 483 au résidu 567, ou à une portion de celle-ci qui est un produit luttant contre le FIV, ou à un variant de celle-ci fonctionnellement équivalent, ou un vecteur d'expression, ou une cellule hôte dans laquelle est insérée une séquence nucléotidique selon l'une quelconque des revendications 1 à 5, pour la stimulation d'une réponse immune dirigée contre les polypeptides codés par la séquence nucléotidique insérée, avec en option, un ou plusieurs adjuvants, et un porteur ou un diluant acceptable du point de vue pharmaceutique.
